# EUROPEAN PATENT APPLICATION

(11) **EP 4 792 726 A1**
(43) Date of publication of application: **19.08.2026**
(21) Application number: 26156971.9
(22) Date of filing: 06.02.2026
(51) Int. Cl.: A61B 1/00, A61B 1/06, A61B 1/07, A61B 1/12, G02B 23/24

(54) **MEDICAL ILLUMINATION DEVICE FOR MEDICAL INSTRUMENT, AND METHOD FOR SUPPLYING A LIGHT BEAM TO A MEDICAL INSTRUMENT**

(30) Priority: 18.02.2025 LU 600261
(71) Applicant: Karl Storz SE & Co. KG, 78532 Tuttlingen (DE)
(72) Inventor: Tischler, Jan-Heiko, 78532 Tuttlingen (DE)

(57) **Abstract**

A medical illumination device (40) and a medical system for supplying a light beam into a medical instrument (10) having a light guide (25), in particular of an endoscopic instrument, comprises a light source unit (44) having at least one light source (46), an optical fiber unit (50) having a light source end (502) connectable to the light source unit (44) and an instrument end (504) connectable to the medical instrument (10), an diaphragm unit (60) moveably arranged between the at least one light source (46) and the optical fiber unit (50), and a drive unit (85) for moving the diaphragm unit (60) relative to the optical fiber unit (50). The diaphragm unit (60) comprises several openings (600) each comprising a different diameter. The optical fiber unit (50) comprises a plurality of optical fibers (510) in an ordered arrangement such that an input end (512) and an output end (514) of the optical fibers are located at equal radial distance to a longitudinal axis (L) of the optical fiber unit (50). The drive unit (85) moves the diaphragm unit (60) such that a selected opening (602; 604; 606; 608) is positioned in a light beam (42) provided by the light source unit (44) for defining a diameter of an input light beam (43) entering the medical instrument (10). A method for supplying a light beam into the medical instrument (10) selects an opening (600) to be moved into the light beam (42) according to at least one operating parameter value of the medical instrument (10) for defining a diameter of the input light beam (43) entering the light guide (10).

## Description

### FIELD OF THE INVENTION

The invention relates to a medical illumination device for supplying a light beam into a medical instrument, particularly an endoscopic instrument, for illuminating an observation area, for example within a body cavity. Further the invention relates to a medical system, such as an endoscopic system, comprising a medical illumination device and at least one medical instrument, and a method for supplying a light beam from the medical illumination device into a medical instrument.

### BACKGROUND OF THE INVENTION

An endoscopic system usually comprises one or more endoscopic instruments and an endoscopic illuminator that can be attached to the endoscopic instrument for illuminating an area of observation. The endoscopic instrument for example includes an endoscopic shaft member attached to a handle or camera head. The endoscopic illuminator can be integrated within or coupled to a scope controller, which also powers and communicates with the endoscopic instrument through an electrical cable. The scope controller provides an image signal to a display or monitor. Light collected by an objective lens at the distal end of the endoscopic shaft is alternatively captured by a, usually distally placed, image sensor within the shaft, or relayed down the length of the shaft via a relay lens system to an image sensor located within the camera head. The distal end of the shaft can be inserted into an otherwise inaccessible space, such as a body cavity accessed through a small incision. An illumination source of the endoscopic illuminator produces illumination light that is directed via an optical fiber to a light post at the endoscope shaft, where the optical shaft is coupled for example with a light guide that carries the illumination light beam to the distal end of the shaft, where it is then able to illuminate a scene within the observation area.

The scope controller and the endoscopic illuminator can be used for various medical instruments of the medical system, such as flexible or rigid endoscopes, imaging endoscopes, laser probes or surgical instruments, like grasping or cutting instrument, etc., Electrical connections of the medical instruments are mostly standardized, and the same electrical cable can be used for coupling the scope controller and the endoscopic illuminator to different medical instruments. However, each of the medical instruments may require different illumination characteristics, for example different wavelength, bandwidth, intensities, polarization, coherence, etc. which determines radiation parameters of a light beam provided for a specific medical instrument. Further, the different medical instruments may be provided with different light guides or light emitting units in their shafts depending on their objective lens systems, light emission requirements and system functionalities. For example, endoscopic instruments may comprise light guides with diameters between around 1 mm and 6mm.

The illumination source of the illuminator is selected to comply with the highest illumination requirements and comprises the highest possible radiation power to ensure compatibility with the various different medical instruments. Consequently, the radiation intensity can be oversized for medical instruments requiring less radiation power, for example instruments with smaller objective systems. If such low radiation instruments are supplied with high radiation power, a large portion thereof is lost at the light entry zone of the medical device, on the way to the distal end of the medical device and at the distal end of the device. The power loss is converted into heat; sometimes over 99% of the radiation energy. For better compatibility, various optical cables are provided for connecting medical instruments with the illuminator, wherein each optical cable is adapted for a specific medical instrument and/or a specific use case of a medical instrument. Particularly, there are optical cables having different diameters corresponding to the light guides or light emitting units of the medical instruments. This may reduce power loss in the medical instrument and shifts the heat development more to light entry zone of the medical device-However, current measurements have shown that there still can be up to 100°C at the connecting parts of the optical cable and the medical instrument and over 50°C at the distal end of the medical instrument. This is particularly the case, when an incorrect optical cable is selected for operating the medical instrument, which can easily happen in situations where several different optical cables are provided for a medical system.

It is known to controll the illumination intensity provided by the illuminator into the medical device by limiting the radiation input into the light guide of the medical device to prevent overheating. US 7,828,726 B2 for example shows an endoscope light source unit for making illumination light incident on a light guide of an endoscope which is disconnectably connected to the endoscope light source unit. The light source unit includes an aperture disc having aperture openings of different opening ratios which are selectively positioned between the incident end face of the endoscope light guide and the light source. The endoscope comprises a memory storing endoscope-type information, for example information on the illumination light quantity limit. A reading device reads the information. A controller selects one aperture opening, an opening ratio of which corresponds to the endoscope-type information, and controlls a drive device to position the selected aperture opening between the incident end face of the light guide and the light source for limiting the light quantity provided to the endoscope. The opening ratios of the different aperture openings are realized by regions in the aperture disc comprising differing density or different numbers of small holes in the disc. This way different opening ratios can be achieved by different hole density/numbers at the aperture regions, functioning like grey filters having differing grey shades, for weaking the illumination intensity entering the endoscope light guide. Consequently, the illumination intensity of light emitted to the observation area also is restricted by the aperture opening dimming the light for illuminating a scene, which may impact the imaging quality of the endoscopic system.

For ensuring high image quality it is known to controll illumination at a scene of the observation area. For example, differing liquid or tissue characteristics show different optical characteristics and require different illumintation for optimal image capturing. For controlling the illumination of the observation area it is known to detect the brightness at an observation scene and provide a feedback signal to an illumination controller for controlling the light source of the illuminator. For example, a camera of the endoscopic instrument may send feedback signals indicating underexposure/overexposure of the observation scene to the controller, which adjusts the light intensity emitted by the light source to optimize the brightness for imaging, as for example described in EP 2457093 B1. The controller may also control a shutter speed according to a light exposure value for preventing overheating and burning of tissue due to a small distance between the distal end of the endoscopic instrument and the observed tissue. Other systems use a diaphragm means positioned between the light source and the light guide of an endoscopic device for luminance adjustment. For example, in US 2008/0278936 A1 a disc-shaped shielding means comprising a cone-shaped radial cutout is used to reduce the light intensity provided for imaging. In WO 2009/090664 A1 the illumination intensity is controlled by a circular shielding disc comprising a circumferential slit of increasing width, wherein the slit opening is filled with a grating of gradually increasing opacity. In such systems a feedback signal providing information about the luminance at the observation scene is provided to the illumination controller for positioning the diaphragm means and adjusting the illumintation for optimal image capturing.

However, systems tuning light radiation towards a scene of the observation area based on brightness at the observation scene may still create light beams of large radiation power which may results in overheating of parts of the medical instrument. It is not taken into account that portions of the radiation power can be lost as heat in or at the light guide of the medical instrument on the way to the observation area. The overheating may harm a patient or phycisian, or may damage components of the instrument.

### SUMMARY OF THE INVENTION

There is a need for a medical illumination device for supplying a light beam into a a medical instrument, particularly of an endoscopic instrument, that can be flexibly adjusted for use in combination with various different medical instruments, that reduces the risk of overheating of components of the medical instrument, that allows for simple and automated adjustment of a light beam spot size provided to the medical instrument, and that is based on a simple setup of a small number of components.

Further, there is a need for a medical system comprising a medical illumination device and at least one medical instrument connectable to the medical illumination device for receiving a light beam that reduces heat loss in components of the medical instrument while still providing a maximal light intensity which can be safely transferred and processed by the medical instrument, that is easily adaptable to characteristics of specific medical instruments, and that allows for simple and reliable assembly and operation.

Further, there is a need for a method for supplying a light beam from a medical illumination device into a medical instrument, that enables automated adaption of radiation power of a light beam provided to a medical instrument, that enables reliable and safe selection of radiation power for avoiding overheating, that allows for improved illumination of an observation area, and that simplifies operation of the medical illumination device.

These and other needs are achieved by a medical illumination device as it is defined by the features of independent claim 1, by a medical system comprising a medical illumination device and at least one medical instrument as it is defined by the features of claim 12 and by a method for supplying a light beam from a medical illumination device into a medical instrument as it is defined by independent claim 13. Preferred embodiments are subject of dependent claims.

According to an aspect of the present invention a medical illumination device for supplying a light beam into a medical instrument, in particular an endoscopic instrument, comprises a light source unit, an optical fiber unit, a diaphragm unit and a drive unit for moving the diaphragm unit relative to the optical fiber unit. The light source unit has at least one light source, for example one or more LED lights, for producing a light beam to be provided to the medical instrument for illuminating an observation scene. The optical fiber unit has a light source end connected or connectable to the light source unit for receiving an input light beam and an instrument end connectable to a light guide of a medical instrument, especially of an endoscopic instrument, for transmitting the input light beam to the medical instrument.

The optical fiber unit comprises a plurality of optical fibers in an ordered arrangement such that an input end and an output end of the optical fibers are located at equal radial distance to a longitudinal axis of the optical fiber unit. For example, the input ends are arranged in an input pattern around the longitudinal axis, and the corresponding output ends are arranged in an output pattern that is the same as the input pattern and may be rotated around the longitudinal axis.

The diaphragm unit is moveably arranged between the at least one light source and the optical fiber unit. Preferably, the diaphragm unit is moveable within a plane perpendicular to the longitudinal axis of the optical fiber unit and keeps the same longitudinal distance to the light source unit and the optical fiber unit. The diaphragm unit comprises several openings each comprising a different diameter. The drive unit is configured to move the diaphragm unit such that a selected opening is positioned in the light beam provided by the light source unit for defining a diameter of the input light beam entering the optical fiber unit, especially for confining the diameter of the input light beam. The drive unit may be operated manually or by a motor such as an electrical motor. Thus, different openings create input light beams of different diameter, particularly of different diameter in a spot size at the light source end of the optical fiber unit. The different openings cut off an outer peripheral light area while the light intensity in an inner light area corresponding to the opening diameter remains the same. Such defined input light beams are incident on the ordered arrangement of optical fibers at the light source end of the optical fiber unit. A number of input ends of optical fibers, particularly a pattern of input ends corresponding to the spot size of the input light beam, are receiving the input light beam. The defined input light beam exits the ordered arrangement of optical fibers at the instrument end of the optical fiber unit with the same diameter and the same light intensity as at the inner light area, wherein the instrument end is connected or connectable to the medical instrument or a light guide thereof for transmitting the defined input light beam.

Advantageously, an opening of the diaphragm unit is selected according to an operating parameter of the medical instrument, such as a diameter size of the light guide of the medical instrument, dimensions of the objective system, or any other structural or operational parameter. Advantageously, the input light beam entering the optical fiber unit and exiting from the instrument end of the optical fiber unit is confined to the light guide diameter size, fiber taper entrance size, the dimensions of the objective system, etc. Accordingly, the input light beam carries the full light intensity that is possible for a specific light guide diameter, fiber taper entrance size, medical instrument design, etc. but excess light that cannot be transferred to the observation area due to the specifics of the medical instrument and that could cause overheating of the medical instrument is eliminated before entering the medical instrument, particularly before entering a light guide of an endoscope. Adapting the diameter of the light beam of the light source unit to characteristics of the medical instrument and transmitting the adapted input light beam via the ordered arrangement of optical fibers reduces heat loss in the medical instrument. At the same time the defined input light beam ensures that a maximum of radiation power is available for operating the medical instrument, wherein the maximum is defined by the specifics of the medical instrument and the entendue effect limiting its capability of processing illumination light. The maximum radiation power of the input light beam may be emitted into the observation area or may be subject of fine tuning for further adaption of the desired illumination conditions at the observation area according to certain applications of the medical instrument, for example by controlling the light intensity emitted by the light source unit.

In one embodiment of the medical illumination device the light source end of the optical fiber unit defines a radially extending light input area including the input ends of the plurality of optical fibers. Preferably, the light beam emitted by the at least one light source is focused and directed such that a light spot of the light beam covers the light input area of the optical fiber unit, i. e. the input ends of the plurality of optical fibers are illuminated by the light beam. Ideally, the light spot of the light beam is not larger than the light input area of the optical fiber unit to avoid loss of radiation. Preferably, a light spot diameter at the light input area corresponds to a diameter of the light input area.

The openings of different diameter of the diaphragm unit correspond to subareas of the light input area of the fiber unit. The subareas include a subset of the plurality of optical fibers. For example, the largest opening may define an input light beam that allows for illuminating the full light input area, i.e. all of the input ends of the optical fiber unit. The openings of decreasing diameter confine the light beam to create input light beams having spot sizes that cover subareas of decreasing size and decreasing number of input ends of optical fibers. That means, by selecting an opening an input light beam entering the optical fiber unit is defined, that may illuminate the full light input area or a subarea of input ends. At the instrument end of the fiber unit a subarea of output ends is equal to the subarea of input ends due to the ordered arrangement of the optical fibers in the optical fiber unit. The subareas may encircle and define the size of the input end patterns. As the aperture opening is selected according to the needs and capabilities of the medical instrument, the subarea is adjusted to such demands as well and the optical fiber output ends of the adjusted subarea feed the defined input light beam into the medical instrument or the light guide thereof. Peripheral areas outside the subareas of the fiber unit are not illuminated and therefore do not transmit radiation that could result in undesired heat development. Nevertheless, the medical instrument receives the maximum illumination intensity because the input ends are illuminated with the maximum radiation power of the light beam created by the light source unit.

According to a further aspect of the present invention a medical system at least comprises a medical illumination device as described above, a controller for controlling at least the medical illumination device, and at least one medical instrument having a light guide. The controller may be a component of the medical illumination device, the medical instrument or a stand-alone controller connected or connectable to the medical illumination device and the medical instrument. The at least one medical instrument is connectable to the medical illumination device for supplying an input light beam into the medical instrument or a light guide thereof. The medical system may include several different medical instruments that can be coupled to the medical illumination device, for example flexible or rigid endoscopes, imaging endoscopes, laser probes or surgical instruments, like grasping or cutting instruments, etc. The medical instrument may be designed, for example, to be introduced at least partially and preferably at least to a large extent into an artificial and/or natural opening, in particular a body opening, in order to carry out a treatment and/or examination in an observation area. An endoscopic instrument can be, for example, an endoscopic forceps instrument, an endoscopic scissors instrument, an endoscopic scalpel instrument, an endoscopic clamp instrument or the like.

For example, the light guide of the medical instrument may be provided in a shaft element of the medical instrument and may guide the input light beam to a lens system as explained earlier for prior art systems. The medical system comprises at least one sensor configured for capturing at least one value of at least one operating parameter of the medical instrument. For example, the sensor is connectable to the controller for automatically controlling the drive unit of the medical illumination device for positioning an opening in a light beam of the light source unit, wherein the opening is selected according to at least one operating parameter and at least one value thereof.

Advantageously, the sensor is an imaging sensor, for example a camera, of the medical instrument, particularly a camera used for endoscopic imaging. Alternatively, the sensor may be any type of parameter sensor, scanner or reader capable of capturing a value of at least one operating parameter of the medical instrument connected to the optical fiber unit of the medical illumination device. The operating parameter may for example characterize a diameter and/or length of the light guide, details of an objective system, physical characteristics of an observation area, imaging parameters such as brightness or contrast at the observation area, etc. Also, the operating parameter may be an identification code representing characteristics of the medical instrument. Advantageously, the at least one sensor automatically captures the one or more parameter values and the controller automatically selects an opening and drives the diaphragm unit to position the selected opening in the light beam.

The input light beam defined by the medical illumination device and supplied to the medical instrument is customized to the needs and capabilities of the medical instrument and the light guide thereof. The input light beam can be flexibly adjusted to these requirements by the medical illumination device while at the same time allowing for a maximum of radiation power for a specific medical instrument and reducing the risk of overheating of the medical instrument. The medical system comprises a simple setup with just one optical fiber unit for various different medical instruments having different light guides. It is easy to be assembled and quickly ready for use.

According to a further aspect of the present invention a method for supplying a light beam from a medical illumination device to a medical instrument is provided. The method comprises a step of emitting a light beam from a light source unit towards an optical fiber unit having a light source end connected or connectable to the light source unit and an instrument end connectable to the medical instrument or a light guide thereof. The method further comprises a step of moving an opening of a diaphragm unit comprising several openings each having a different diameter into the light beam in front of the optical fiber unit for defining an input light beam. The method further comprising feeding the defined input light beam into optical fibers of the optical fiber unit, wherein the optical fibers are arranged in an ordered arrangement such that an input end and an output end of the optical fibers are located at equal radial distance relative to a longitudinal axis of the optical fiber unit. The method further comprises a step of selecting an opening to be moved into the light beam according to operating parameter values of the medical instrument for defining the diameter of the input light beam entering the optical fiber unit.

Advantageously, the method uses the medical illumination device and is executed by a medical system as described above.

In a variant of the method for supplying a light beam the step of feeding the defined input light beam into the optical fiber unit comprises feeding the input light beam to the subarea of the radially extending light input area including the input ends of the optical fibers of the optical fiber unit. The subarea includes the subset of optical fibers and is defined by the selected opening, which means it is defined by the spot size limited by the opening.

In one example embodiment of the medical illumination device the several openings of the diaphragm unit are circular openings, like aperture openings. The circular shaped openings confine the light beam to a circular spot size on the light input area of the optical fiber unit. They also define circular subareas of the light input area. A circular shape of the openings and the subareas optimizes the transmission of radiation power into the optical fiber unit and into the medical instrument. Advantageously, a center of the openings is located on the longitudinal axis of the optical fiber unit. As a result, the subareas of illuminated input ends are concentric areas with their centers on the longitudinal axis. When changing between openings to create differently sized defined input light beams, the inner most optical fiber or core fibers is consistently illuminated and rings of input ends around the core fiber are added or subtracted according to the diameter of the openings. This way, the center of the input light beam is the same for all openings and the center of illumination at the output areas is at the same spot independent of the type of medical instrument or light guide thereof. A light post or connector used for connecting for example to the light guide of the medical instrument aligns the axis of the light guide with the longitudinal axis of the optical fiber unit. It is simple to switch between different medical instruments, respectively light guides, and comply with their differing requirements by changing the opening. No additional alignment or components are needed to prepare the medical system for use.

In a further embodiment of the medical illumination device the optical fibers are arranged parallel and/or spiral along the longitudinal axis of the optical fiber unit, wherein the input ends and the output ends of the optical fibers are located at equal radial distance to the longitudinal axis to ensure the ordered arrangement of the optical fibers. For example, the input ends and the output ends of the optical fibers can be arranged at the same circumferential position of the longitudinal axis. They are located at the same circumferential coordinates on an input plane and on an output plane that are perpendicular to the longitudinal axis. The fibers can all be arranged parallel or all be arranged spiraling around the axis. Also, a core can have parallel fibers and spiraled fibers around them or the other way round. The parallel or spiral alignment of the optical fibers supports enlarging or reducing the subset of optical fibers and the illuminated subarea, respectively, by adding or removing rings of input ends around the core fibers. A parallel and/or spiral arrangement of optical fibers allows for bundling the radiation power provided by the input light beam along the longitudinal axis and avoids loss of light intensity at the instrument end of the optical fiber unit. Further, the plurality of optical fibers could be arranged in a tapered structure at the light source end and a corresponding tapered structure at the instrument end, such that the input ends and the output ends of the optical fibers are located at equal radial distance to the longitudinal axis to ensure the ordered arrangement of the optical fibers. Between the tapered ends the optical fiber unit comprises a smaller diameter as at their ends. The tapered ends facilitate a connection of the optical fiber unit to the light source unit and the medical instrument or light guide thereof.

In one example embodiment of a diaphragm unit, it may be realized as a rotatable disc and the several openings are arranged on a concentric circumferential line around a rotation axis according to increasing opening diameters. The centers of the openings are positioned on the concentric line. Advantageously, the rotation axis is offset and parallel to the longitudinal axis of the optical fiber unit and the concentric line crosses the longitudinal axis. The rotatable diaphragm disc is rotated around the rotation axis by the drive unit to position the openings in front of the light input area of the optical fiber unit. This set up allows for fast changing of the openings and adapting the input light beam.

In another example embodiment of a diaphragm unit, it may be realized as a linearly slidable slider and the several openings are arranged on a straight line according to increasing opening diameters. Advantageously, the straight line is arranged perpendicular to and crosses the longitudinal axis of the optical fiber unit. The diaphragm slider is moved linearly by the drive unit relative to the longitudinal axis to position the openings in front of the light input area of the optical fiber unit. This set up also allows for fast changing of the openings and adapting the input light beam.

In a further example embodiment of a diaphragm unit, it may comprise a solid surface section having a diameter at least equal to the diameter of the optical fiber unit, particularly equal to the diameter of the light input area. The solid surface section does not have any openings or through holes. It may absorb or scatter the light beam away from optical fiber unit. The solid surface section of the diaphragm unit serves as a mechanical light valve to shut off light towards the optical fiber unit. It helps protecting the medical instrument and the observation area from excessive radiation power for example at the beginning of the adjustment of the input light beam.

In a further embodiment the medical illumination device may comprise a controller for automatically controlling the drive unit for positioning the openings of the diaphragm unit into the input light beam. The drive unit may be electrically connected to the controller. The controller may be configured for capturing and/or receiving an electronic signal representing an operating parameter value of the medical instrument. Further the medical illumination device may comprise a processing unit for automatically selecting an opening to be moved into the light beam according to operating parameter values of the medical instrument. The processing unit may be part of the controller. Further the controller may comprise a memory, an image processing module, a camera control unit, and further electronic components as needed for operating the medical illumination device and the medical system, particularly as needed for executing the method for supplying the defined input light beam from the medical illumination device into the medical instrument or the light guide thereof.

In a variant of the method for supplying a light beam the step of selecting the opening for supplying the input light beam from the medical illumination device into the medical instrument comprises a step of subsequently moving a series of openings into the light beam starting from a small diameter opening and subsequently progressing to larger diameter openings. A further step comprises capturing an operating parameter value of the medical instrument for each opening of the series of openings, and identifying the smaller diameter opening of two subsequent openings showing the same operating parameter value or at least insignificantly different values. A further step comprises determining the smaller diameter opening as the selected opening to be moved into the light beam for providing the defined input light beam for entering the optical fiber unit.

For example, the sensor detects a change in image brightness while increasing the opening diameter. In case there is no change in brightness, respectively no significant change in brightness, between a preceding opening of smaller diameter and a successive opening of larger diameter the controller selects the preceding opening for defining the input light beam to be provided to the medical instrument. This way, an optimum spot size can be determined, where no excess light is coupled into the medical instrument when operating the medical instrument. Using an opening with a larger diameter, i.e. providing higher radiation power to the medical instrument, would only lead to a temperature increase in the light guide and the objective system and especially in the medical instrument because the radiation cannot be transmitted to the observation area due to entendue conservation and the properties of the medical instrument.

Advantageously, the step of selecting the diaphragm opening may comprise automatically moving the series of openings into the input light beam and automatically identifying the smaller diameter opening of two subsequent openings after connecting the medical instrument or the light guide thereof to the optical fiber unit of the medical illumination device. For example, a specific medical instrument is connected to the medical illumination device and the controller initiates the automatic identification of an optimal opening when the light source unit is turned on. The drive unit controlled by the controller rotates or slides the diaphragm unit for subsequently moving openings of increasing diameter into the light beam. The sensor, such as the camera of the medical instrument, automatically acquires values of an operating parameter, e.g. the image brightness, and transmits a value signal to the controller or processing unit, which selects the smaller of two subsequent openings that both provide the same or similar parameter value, e.g. a maximum image brightness, as the selected optimal opening for the specific medical instrument and the specific observed scene. For the selection of one of two subsequent openings, their respective parameter values ideally indicate that a difference in illumination quality or imaging quality between the two openings is insignificant or neglectable. The parameter values of two subsequent openings shall for example be regarded as sufficiently similar, when a difference between the values is below 5%, preferably below 3%.

The automatic identification of the optimal diaphragm opening provides a quick and easy process to prepare the medical instrument for use. It guarantees safe operations of the medical instrument by reducing the risk of overheating while it does not limit the technical capabilities of the medical instrument. The method provides the maximal processable radiation power to the observation area. It allows the medical system to provide high quality illumination of an observed scene as a basis for fine tuning the illumination level at the observation area for example by controlling the light source of the medical illumination device.

### BRIEF DESCRIPTION OF THE DRAWING

- Figure 1: shows a schematic illustration of a medical system comprising a medical illumination device and a medical instrument according to the invention,
- Figure 2: shows a schematic diagram of the medical illumination device according to the invention,
- Figure 3a: shows a schematic diagram of a first embodiment of a diaphragm unit of the medical illumination device of the present invention,
- Figure 3b: shows a schematic diagram of a second embodiment of a diaphragm unit of the medical illumination device of the present invention, and
- Figure 4: shows a schematic block diagram illustrating steps of a variant of a method for supplying a light beam from a medical illumination device into a light guide of a medical instrument according to the invention.

### DETAILED DESCRIPTION OF PREFERRED EMBODIMENTS

The detailed description is set forth with reference to the accompanying drawings. The drawings are provided for purposes of illustration only and merely depict example embodiments of the disclosure. The drawings are provided to facilitate understanding of the disclosure and shall not be deemed to limit the breadth, scope, or applicability of the disclosure. Various embodiments may utilize elements or components other than those illustrated in the drawings, and some elements and/or components may not be present in various embodiments.

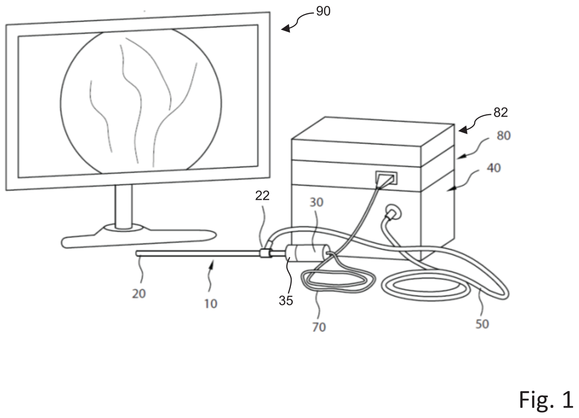

FIG. 1 illustrates a medical system with a medical instrument 10 and a medical illumination device 40 according to an example embodiment of the invention. The example medical instrument 10 is an endoscopic instrument which comprises an endoscopic shaft 20 attached to a handle or camera head 30. The medical illumination device 40, a controller 80 and a processing unit 82 may be at least partially integrated as depicted within a common rack. The controller 80 powers and communicates with the medical instrument 10 through an electrical cable 70 and is electrically connected to the medical illumination device 40.

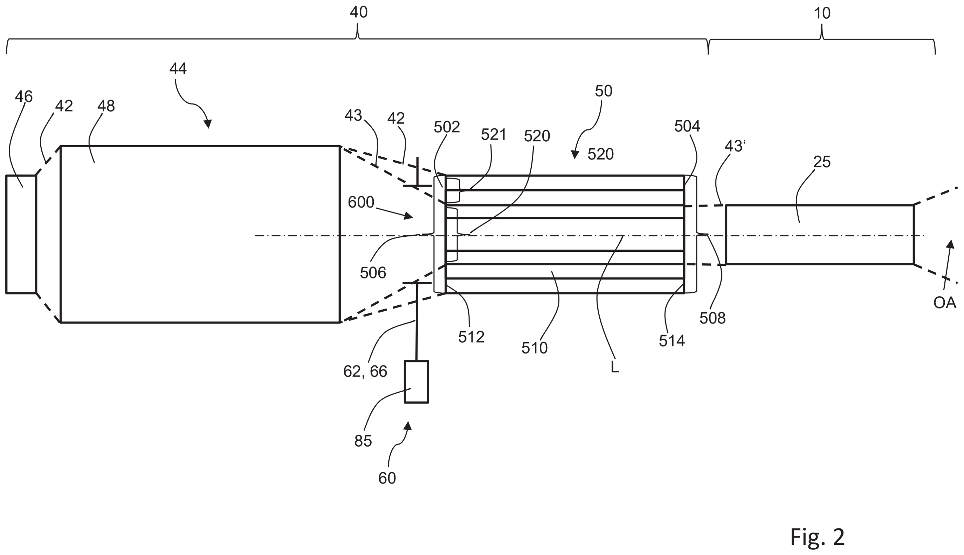

The medical illumination device 40 comprises an elongated flexible optical fiber unit 50. One end of the optical fiber unit 50 is connectable to a light guide 25 of the medical instrument 10 arranged in the endoscopic shaft 20 by a light post 22. In other variants of a medical instrument, the optical fiber unit 50 may for example be connected directly to the medical instrument, to a light emitting unit thereof, or similar component serving as a light guide. The other end is connectable to a light source unit 44 of the medical illumination device 40. The medical illumination device 40 produces an input light beam 43 as will be explained with reference to Figure 2. The input light beam 43 is directed into the optical fiber unit 50 which carries the input light beam to the light post 22 and the light guide 25 that carries the input light beam to the distal end of the shaft 20. Light collected by an objective lens system at the distal end of the endoscopic shaft 20 is alternatively captured by a, usually distally placed, image sensor 35 within the shaft, or relayed down the length of the shaft via a relay lens system to an image sensor 35' located within the camera head. The distal end of the shaft 20 is inserted into an otherwise inaccessible observation area, such as in a body cavity accessed through a small incision. The input light beam exits the distal end of the shaft 20 and illuminates the observation area. The controller 80 receives imaging signals from the image sensor, which are processed by the processing unit 82. The controller 80 provides an image signal to a display 90 showing a scene of the observation area OA.

The medical system may include various other medical instruments for various other applications, which may alternatively be coupled to the optical fiber unit 50 of the medical illumination device 40 by the light post 22 and to the controller 80 via the camera head 30 and/or an electrical cable. The various medical instruments may have differing characteristics and different illumination needs at the observation site. The medical illumination device 40 can easily adapt the input light beam 43 according to individual characteristics of specific medical instruments and the light guides thereof for minimizing a risk of overheating the medical instrument or scenes in the observation area OA, as is disclosed below without needing to change further components such as the optical fiber unit 50.

Figure 2 shows a schematic diagram of the medical illumination device 40, wherein units of the medical illumination device 40 and the medical instrument 10 are lined up along a straight line for simplicity. It will be understood that such units can be positioned otherwise than in a straight line due to the flexibility of the optical fiber unit 50, the electrical cable 70 and potentially the medical instrument 10. The medical illumination device 40 is designed for supplying the input light beam 43 into the light guide 25 of the medical instrument 10. It comprises a light source unit 44, the optical fiber unit 50, a diaphragm unit 60 and a drive unit 85 for moving the diaphragm unit 60 relative to the optical fiber unit 50. For example, the drive unit 85 may be a hydraulic, electrical, piezoelectric or pneumatic drive, for mechanically moving the diaphragm unit 60. Alternatively, a manually operated drive unit could be used.

The light source unit 44 has at least one light source 46 emitting light into an illumination system 48 of the light source unit 44 for creating and directing a light beam 42 in direction of the optical fiber unit 50. The light source 46 may be realized as an LED, for example. Optionally, the light source unit 44 may comprise tuning means for tuning the light beam 42. Also, the controller may be configured to control tuning of the light beam 42.

The optical fiber unit 50 has a light source end 502 connected or connectable to the light source unit 44 and an instrument end 504 connectable, for example by the light post 22, to a medical instrument or a light guide thereof, such as the light guide 25 of the medical instrument 10. The optical fiber unit 50 comprises a plurality of optical fibers 510 along a longitudinal axis L, wherein only a few fiber strings are schematically depicted in Figure 2. For example, the optical fibers 510 are arranged parallel and/or spiral along the longitudinal axis L. The optical fibers 510 could be arranged in a tapered structure at the light source end 502 and a corresponding tapered structure at the instrument end 504 (not shown in Figure 2). The plurality of optical fibers 510 are in an ordered arrangement such that an input end 512 and an output end 514 of each of the optical fibers 510 is located at equal radial distance to the longitudinal axis L. The light source end 502 defines a radially extending light input area 506 including all the input ends 512 of the plurality of optical fibers 510. The instrument end 504 defines a radially extending light output area 508 including all the output ends 514. The ordered arrangement of the optical fibers 510 results in an input end pattern and an output end pattern, wherein each of the input ends 512 and the associated output ends 514 have the same radial coordinate relative to the longitudinal axis L in an input plane defined by the light input area 506, respectively an output plane defined by the light output area. Thus, the input ends 512 and the output ends 514 have the same distance to the longitudinal axis L. They may also have the same circumferential coordinate in the input plane and the output plane. Thus, the input ends 512 and the output ends 514 may be arranged at the same circumferential position relative to the longitudinal axis L in the light input area 506 and the light output area 508.

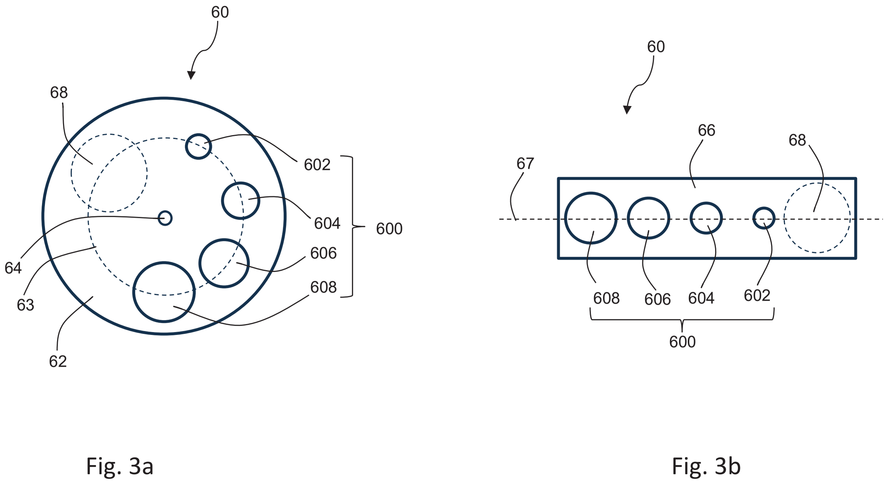

The diaphragm unit 60 is moveably arranged between the at least one light source 46, respectively the illumination system 48, and the optical fiber unit 50. For example, the diaphragm unit 60 is mounted in the light source unit 44 or in the rack as shown in Figure 1, near the light source end 502 of the optical fiber unit 50, preferably as close as possible to the light input area 506. As shown in Figures 3a and 3b, the diaphragm unit 60 comprises several circular openings 600, wherein each opening 602, 604, 606, 608 comprises a different diameter. The drive unit 85 may be controlled by the controller 80 for moving and positioning the openings 600 into the light beam 42 provided by the light source unit 44 for defining a diameter of an input light beam 43 entering the optical fiber unit 50. The largest opening 608 may define a spot size of the input light beam 43 such that the input light beam 43 illuminates the complete light input area 506, i.e. all of the plurality of optical fibers 510, of the optical fiber unit 50. Smaller openings, such as the openings 602, 604 and 606, may confine the light beam 42 to realize a smaller input light beam 43.

A specific opening 602, 604, 606 or 608, i.e. a specific opening diameter, is selected such that excess radiation power that would get lost in the medical instrument 10 due to the entendue effect is excluded from the input light beam 43. Additionally, the opening is selected such that a maximum radiation power that is processable by the medical instrument 10 is transferred by the input light beam 43 to the medical instrument 10. The opening diameter may be selected according to one or more operating parameters of the medical instrument 10, such as a diameter size of the light guide 25, dimensions of the illumination system 48, or any other structural or operational parameter. As mentioned above, different medical instruments comprise different operating parameters and therefore require different input light beams 43 to ensure safe operations without overheating. Preferably, an opening to be moved into the light beam 42 is selected automatically, as will be explained below.

The light input area 506 is illuminated by the input light beam 43. If the largest opening 508 is selected, the light beam 43 is incident on all the input ends 512 of the plurality of optical fiber 510. If a smaller opening 602, 604 or 606 is selected, only a subarea 520 of the light input area 506 is illuminated, wherein the diameter of the subarea 520 corresponds to the diameter of the selected opening. A peripheral area 521 around the subarea 520 does not receive any illumination light. Accordingly, a subset of optical fibers 510 located in the subarea 520 are receiving the defined input light beam 43. The subset of optical fibers 510 corresponds to the spot size of the input light beam 43, i.e. to the diameter of the selected opening. Due to the ordered arrangement of the optical fibers 510, the input light beam 43' exits the subset of optical fibers at the light output area 508 with the same diameter, i.e. with the same spot size and the same light intensity, at the instrument end 504 of the fiber unit 50. Advantageously, a center of the openings 600 is located on the longitudinal axis L of the optical fiber unit 50. Accordingly, the subareas 520 defined by different openings 500 are concentric to the longitudinal axis L.

The size of the subset of optical fibers 510, respectively of the exiting input light beam 43', is optimized according to the capability of the medical instrument 10 to process incoming illumination light, particularly it is adapted according to one or more operating parameters of the medical instrument 10. The optimized input light beam 43' enters the light guide 25 of the medical instrument 10. The concentric arrangement of the subareas 520 facilitates feeding the input light beam 43' into the light guide 25. Optionally, the longitudinal axis L of the optical fiber unit 25 aligns with an axis of the light 25 after coupling them together. The medical instrument 10 receives the maximum radiation power customized for its specific characteristics. For other medical instruments comprising other characteristics different openings 600 may be selected, resulting in different illuminated subareas and different subsets of optical fibers transmitting an input light beam 43 of different maximum radiation power. The optical fiber unit 50 does not need to be exchanged or uncoupled.

Adapting the diameter of the light beam 42 of the light source unit 44 to characteristics of a medical instrument and transmitting an adapted input light beam 43 via the ordered arrangement of optical fibers reduces the risk of overheating of the light guide 25 and the medical instrument. At the same time the defined input light beam 43 ensures that a maximum of radiation power is available for operating the medical instrument and can be emitted into the observation area OA. Optionally, the controller may control the at least one light source 46 and/or the illumination system 48 for fine tuning the illumination conditions at the observation area OA according to specifics of the application of the medical instrument 10.

Figures 3a and 3b show different example embodiments of the diaphragm unit 60. In the example of Figure 3a, the diaphragm unit 60 comprises a rotatable disc 62 and the several openings 600 are arranged on a concentric circumferential line 63 around a rotation axis 64. The openings 602, 604, 606, and 608 are lined up as a series of openings with their centers on the concentric line 63 according to increasing opening diameters. The rotation axis 64 is arranged offset of the longitudinal axis L such that the concentric line 63 crosses the longitudinal axis L of the optical fiber unit 50. In the example of Figure 3b the diaphragm unit 60 comprises a linearly slidable slider 66. The openings 602, 604, 606, and 608 are arranged on a straight line 67 in series according to increasing opening diameters. The slider 66 is arranged such that the straight line 67 crosses the longitudinal axis L and is slidable perpendicular thereto. It will be understood that the several openings 600 may include more than the four openings 602, 604, 606, and 608 depicted in the example embodiments of Figures 3a and 3b. By rotating the rotatable disc 62, respectively sliding the slider 66, the openings can be positioned precisely in the light beam 42 for defining the input light beam 43. The openings can be changed quickly for preparing the medical illumination device 40 for a specific medical instrument.

The diaphragm unit 60 may comprise a solid surface section 68 having a diameter at least equal to the diameter of the optical fiber unit 50, particularly of the light input area 506. The solid surface section 68 is a closed surface area of the rotatable disc 62, respectively the slider 66. By positioning the solid surface section 68 the light beam 42 is blocked in direction of the optical fiber unit 50. The solid surface section 68 serves as a light valve to shut off light towards the medical instrument 10.

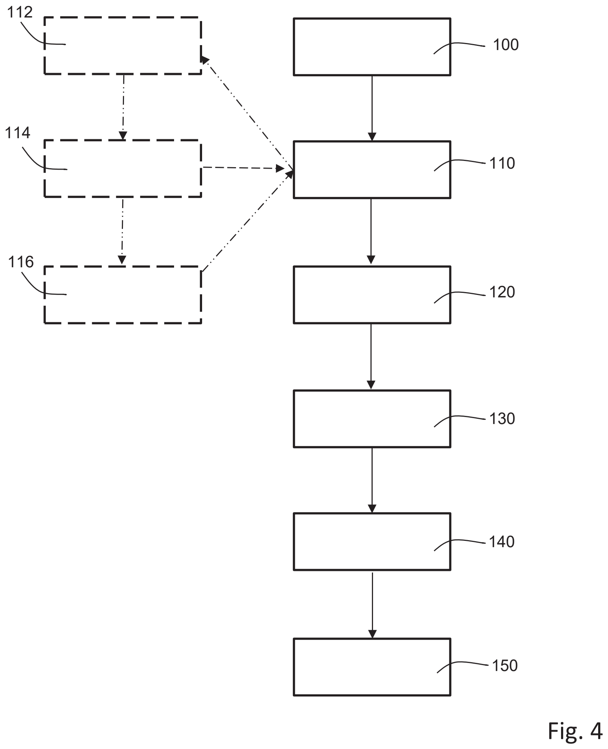

Figure 4 shows a schematic block diagram illustrating method steps of a method variant for supplying an input light beam 43 from a medical illumination device 40 into a light guide 25 of the medical instrument 10. The method will be explained with reference to the medical system described above. Some steps are performed simultaneously or during a period spanning several steps while other steps are sequential as will be explained.

In a light emitting step 100 the light beam 42 is emitted from the light source unit 44 towards the optical fiber unit 50. The light emitting step 100 may include initiating the at least one light source 46, shaping and directing the light beam 42 towards the optical fiber unit 50 as well as optionally controlling a light intensity.

In a selection step 110 an opening of the several openings 602, 604, 606 and 608 of the diaphragm unit 60 is selected, which serves for defining the input light beam 43 used for operating the medical instrument 10 and which is moved into the light beam 42 in a moving step 120. The opening is selected according to at least one value of at least one operating parameter of the medical instrument 10 for defining the diameter of the input light beam 43 entering the optical fiber unit in a feeding step 130. The operating parameter can be an instrument information like an objective lens setup, a diameter/length of the light guide, or an observation area parameter, or an imaging parameter, or any other operational or structural parameter. The selection of the opening may be based on more than one parameter value of the same or different parameter type. The operating parameter value may be provided manually, e.g. entered as data into the controller 80, for selecting an opening to be moved into the light beam 42 and defining the input light beam 43.

In the feeding step 130, the defined input light beam 43 enters the optical fibers 510 of the optical fiber unit 50. Particularly, the input light beam 43 illuminates a subarea 520 of the light input area 506 and enters a subset of input ends 512 according to the spot size defined by the selected opening moved into the light beam 42.

In a supply step 140, the input light beam 43' exiting the optical fibers 510 enters the light guide 25 which supplies the input light beam 43' to the medical instrument 10 for illuminating the observation area OA. The input light beam 43' is optimized for the medical instrument 10. No excess radiation that could lead to overheating of the light guide 25 or the medical instrument 10 is supplied to the medical instrument 10.

Preferably, the selection step 110 may include a value capturing step 114 (as indicated as a dashed line in Figure 4) for capturing a value of at least one operating parameter of the medical instrument 10 by a sensor, especially the imaging sensor 35 of the medical instrument 10. For example, in the value capturing step 114 the imaging sensor 35 captures an imaging parameter value as a operating parameter value of the medical instrument such as a value of the image brightness at a scene observed with the medical instrument.

Advantageously, the selection step 110 includes a subsequent moving step 112 (as indicated as a chain line in Figure 4) for subsequently moving the series of openings 602, 604, 606 and 608 into the light beam 42 starting from the small diameter opening 602 and subsequently progressing to larger diameter openings 604, 606 and 608. For each of the openings 602, 604, 606 and 608 of the series of openings, at least one operating parameter value is captured as described for the value capturing step 114. In an identification step 116 (as indicated as a chain line in Figure 4), the smaller diameter opening of two subsequent openings showing the same or similar operating parameter value, i.e. an at least not insignificantly different value, is identified by the processing unit 82 and determined as the selected opening to be moved into the light beam 42 in the opening moving step 120 for providing the defined input light beam 43 for entering the optical fiber unit 50 and entering the light guide 25 as the light beam 43' exiting the optical fiber unit 50. The processing unit 82 may include a comparison module for comparing parameter values and providing a control signal to the controller 80 that initiates moving to a larger opening or moving back to a previous opening, that has been identified as the selected opening.

Preferably, the controller of the medical illumination device 40 is configured for automatically controlling the drive unit 85 for positioning the series of openings 602, 604, 606 and 608 of the diaphragm unit into the light beam 42 in the moving step 110. Further, the controller is configured for automatically capturing and/or receiving an electronic signal representing an operating parameter value of the medical instrument in the value capturing step 114. The processing unit 82 is configured for automatically selecting an opening to be moved into the light beam 42 according to the operating parameter values of the medical instrument in the identification step 116. Especially, the processing unit 82 is configured for automatically identifying the smaller diameter opening of two subsequent openings, after connecting the light guide 25 of the medical instrument 10 to the optical fiber unit 50 of the medical illumination device 40. As a result, the method for supplying the input light beam 43 into the light guide 25 of the medical instrument 10 is executed fully automatically and no further action is needed to prepare the medical instrument 10 for application at the observation area.

Optionally, in a tuning step 150 the light beam 42 incident to the optical fiber unit 50 may be fine tuned according to operational demands of the medical instrument 10, particularly according to needs of an imaging operation. Advantageously, the medical illumination device 40 provides the maximum safely applicable radiation power as input light beam 43 as a basis for the operation. Fine tuning of the light intensity may improve imaging quality such as contrast, colors, etc.

After supplying the input light beam 43' to the medical instrument 10 and the observation area OA, respectively, and optionally tuning the input light beam 43' for specific demands of the medical instrument 10, the observation area OA and/or specific imaging methods, the medical system is ready for use as known to a skilled person.

The controller 80 and/or the processing unit 82 may comprise at least one computer program to be executed to implement the method as described above. The computer program may be stored/distributed on a suitable medium, such as an optical storage medium or a solid-state medium supplied together with the controller 80 or processing unit 82 or as part of other hardware. Furthermore, the computer program can also be a data structure or a signal for embodying a specific method step such as the method steps according to the invention.

The medical illumination device 40 for supplying an input light beam 43 into the light guide 25 of a medical instrument, particularly of an endoscopic instrument, is flexibly adjusted for use with various medical instruments. It allows for simple and automated adjustment of a light beam spot size provided to the medical instrument for reducing the risk of overheating of components of the medical instrument. The medical illumination device 40 has a simple setup and a small number of components. The medical system comprising the medical illumination device 40 diminishes heat loss in components of the medical instrument 10 while still providing a maximal light intensity which can be safely transferred and processed by the medical instrument 10. The system is easily adaptable to characteristics of specific medical instruments and allows for simple and reliable assembly and operation.

The method for supplying the light beam from the medical illumination device 40 into the light guide 25 of a medical instrument enables automated adaption of radiation power of the input light beam 43 provided to a specific medical instrument. It enables reliable and safe selection of radiation power for avoiding overheating and allows for improved illumination of an observation area. Overall, the method simplifies operation of the medical system.

This description and the accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. Features and properties described in relation to the medical illumination device and the medical system, shall be considered to be disclosed for the method as well and vice versa. This means that structural features, i.e. device-related features, mentioned in relation to methods can also be considered, claimed and counted as part of the disclosure within the scope of the device claims.

### Reference Signs

- 10: medical instrument
- 20: endoscopic shaft
- 22: light post
- 25: light guid
- 30: camera head
- 35: imaging sensor
- 40: medical illumination device
- 42: light beam
- 43, 43': input light beam
- 44: light source unit
- 46: light source
- 48: illumination system
- 50: optical fiber unit
- 60: diaphragm unit
- 62: rotatable disc
- 63: concentric circumferential line
- 64: rotation axis
- 66: linear slider
- 67: straight line
- 68: solid surface sector
- 70: electrical cable
- 80: controller
- 82: processing unit
- 85: drive unit
- 90: display
- 100: light emitting step
- 110: selection step
- 112: subsequent moving step
- 114: value capturing step
- 116: identification step
- 120: opening moving step
- 130: feeding step
- 140: supply step
- 150: tuning step
- 502: light source end
- 504: instrument end
- 506: light input area
- 508: light output area
- 510: optical fibers
- 512: input end of optical fiber
- 514: output end of optical fiber
- 520: subarea
- 521: peripheral area
- 600: openings
- 602: opening
- 604: opening
- 606: opening
- 608: opening

- L: longitudinal axis
- OA: observation area

## Claims

1. Medical illumination device (40) for supplying a light beam into a medical instrument (10) having a light guide (25), in particular of an endoscopic instrument, the medical illumination device (40) comprising:
a light source unit (44) having at least one light source (46),
an optical fiber unit (50) having a light source end (502) connected or connectable to the light source unit (44) and an instrument end (504) connectable to the light guide (25) of the medical instrument (10),
a diaphragm unit (60) moveably arranged between the at least one light source (46) and the optical fiber unit (50), and
a drive unit (85) for moving the diaphragm unit (60) relative to the optical fiber unit (50), wherein the diaphragm unit (60) comprises several openings (600) each comprising a different diameter,
wherein the optical fiber unit (50) comprises a plurality of optical fibers (510) in an ordered arrangement such that an input end (512) and an output end (514) of the optical fibers are located at equal radial distance to a longitudinal axis (L) of the optical fiber unit (50), and
wherein the drive unit (85) is configured to move the diaphragm unit (60) such that a selected opening (602; 604; 606; 608) is positioned in a light beam (42) provided by the light source unit (44) for defining a diameter of an input light beam (43) entering the optical fiber unit (50).

2. Medical illumination device (40) according to claim 1, wherein the light source end (512) of the optical fiber unit (50) defines a radially extending light input area (506) including the input ends (512) of the plurality of optical fibers (510), and openings (602; 604; 606; 608) of different diameter correspond to subareas of different diameter including a subset of the plurality of optical fibers (510).

3. Medical illumination device (40) according to claim 1 or 2, wherein the several openings (600) of the diaphragm unit (60) are circular openings.

4. Medical illumination device (40) according to any of the preceding claims, wherein a center of the openings (600) is located on the longitudinal axis (L) of the optical fiber unit (50).

5. Medical illumination device (40) according to any of the preceding claims, wherein the optical fibers (510) are arranged parallel and/or spiral along the longitudinal axis (L) of the optical fiber unit (50).

6. Medical illumination device (40) according to any of the preceding claims, wherein input ends (512) and output ends (514) of the optical fibers (510) are arranged at the same circumferential position of the longitudinal axis (L).

7. Medical illumination device (40) according to any of the preceding claims, wherein the diaphragm unit (60) comprises a rotatable disc (62) and the several openings (600) are arranged on a concentric circumferential line (63) around a rotation axis (64) of the rotatable disc (62) according to increasing diameters of the openings (602; 604; 606; 608).

8. Medical illumination device (40) according to any of the preceding claims 1-6, wherein the diaphragm unit (60) comprises a linearly slidable slider (66) and the several openings (600) are arranged on a straight line according to increasing diameters of the openings (602; 604; 606; 608).

9. Medical illumination device (40) according to any of the preceding claims, wherein the diaphragm unit (60) comprises a solid surface section (68) having a diameter at least equal to the diameter of the optical fiber unit (50).

10. Medical illumination device (40) according to any of the preceding claims, comprising a controller (80) for automatically controlling the drive unit (85) for positioning the openings (600) of the diaphragm unit (60) into the light beam (42).

11. Medical illumination device (40) according to any of the preceding claims, comprising a controller (80) for capturing and/or receiving an electronic signal representing an operating parameter value of the medical instrument (10) and a processing unit (82) for automatically selecting an opening (602; 604; 606; 608) to be moved into the light beam (42) according to at least one operating parameter value of the medical instrument (10).

12. Medical system comprising a medical illumination device (40) according to one of the claims 1-11 and at least one medical instrument (10) having a light guide (25), wherein the light guide (25) of the medical instrument (10) is connectable to the medical illumination device (40) for supplying an input light beam (43) into the light guide (25),
wherein the medical system comprises at least one sensor (35) configured for capturing a value of at least one operating parameter of the medical instrument (10),
wherein the sensor (35) is connected or connectable to a controller (80) for controlling the drive unit (85) of the medical illumination device (40) and positioning an opening (602; 604; 606; 608) in a light beam (42) of the light source unit (44), and
wherein the opening (602; 604; 606; 608) is selected according to at least one value of at least one operating parameter.

13. Method for supplying a light beam from a medical illumination device (40) into a light guide (25) of a medical instrument (10), comprising:
emitting (100) a light beam (42) from a light source unit (44) towards an optical fiber unit (50) having a light source end (502) connected or connectable to the light source unit (44) and an instrument end (504) connectable to the light guide (25) of the medical instrument (10),
moving (120) an opening of a diaphragm unit (60) comprising several openings (602; 604; 606; 608) each having a different diameter into the light beam (42) in front of the optical fiber unit (50) for defining an input light beam (43),
feeding (130) the defined input light beam (43) into optical fibers (510) of the optical fiber unit (50), wherein the optical fibers (510) are arranged in an ordered arrangement such that an input end (512) and an output end (512) of the optical fibers (510) are located at equal radial distance to a longitudinal axis (L) of the optical fiber unit (50),
selecting (110) an opening (600) to be moved into the light beam (42) according to at least one operating parameter value of the medical instrument (10) for defining a diameter of the input light beam (43) entering the optical fiber unit (50), and
feeding the input light beam (43') exiting the optical fiber unit (50) into the light guide (25).

14. Method of claim 13, wherein feeding (130) the defined input light beam (43) into the optical fiber unit (50) comprises:
feeding the input light beam (43) to a subarea (508) of a radially extending light input area (506) including the input ends (512) of the optical fibers (510) of the optical fiber unit (50), wherein the subarea (508) includes a subset of optical fibers and is defined by the selected opening (602; 604; 606; 608).

15. Method of claim 13 or 14, wherein selecting (110) the opening (600) for supplying the input light beam (43) from the medical illumination device (40) into the medical instrument (10) comprises:
subsequently moving (112) a series of openings (602, 604, 606, 608) into the light beam (42) starting from a small diameter opening and subsequently progressing to larger diameter openings,
capturing (114) an operating parameter value of the medical instrument (10) for each opening of the series of openings (602, 604, 606, 608),
identifying (116) the smaller diameter opening of two subsequent openings showing similar operating parameter value, and
determining the smaller diameter opening as the selected opening to be moved into the light beam (42) for providing the defined input light beam (43) for entering the optical fiber unit (50).

16. Method of the claim 15, wherein selecting (110) the opening comprises:
automatically moving the series of openings (602, 604, 606, 608) into the input light beam (42) and automatically identifying the smaller diameter opening of two subsequent openings, after connecting the medical instrument (10) to the optical fiber unit (50) of the medical illumination device (40).

17. Method of one of claims 13 - 16, wherein selecting (110) the opening (600) comprises:
capturing (114) an imaging parameter value by an image sensor (35) of the medical instrument (10) representing a value of the operating parameter of the medical instrument (10), particularly a value of an image parameter.
